# EUROPEAN PATENT APPLICATION

(11) **EP 2 564 816 A2**
(43) Date of publication of application: **06.03.2013**
(21) Application number: 12183549.0
(22) Date of filing: 16.02.2006
(51) Int. Cl.: A61F 2/60, A61F 2/68

(54) **System and method of synchronizing and communicating with mechatronic devices**

(30) Priority: 15.02.2006 US 355047; 16.02.2005 US 653717 P; 10.05.2005 US 679953 P
(62) Divisional of application: 06720786.0
(71) Applicant: Ossur HF, 110 Reykjavik (IS)
(72) Inventor: Clausen, Arinbjörn, V., IS-108 Reykjavik (IS); Oddsson, Magnus, 110 Reykjavik (IS)
(74) Representative: Brand, Thomas Louis

(57) **Abstract**

A system for controlling motion of a human limb may include a plurality of mechatronic devices (202,204), each of which may be in communication with at least one other of the plurality of mechatronic devices. Each of the mechatronic devices includes one or more of a processor (360), an actuator, or a sensor. One or more of the mechatronic devices may be configured to generate a control state for at least one other of the plurality of mechatronic devices based on the communicated data. In one embodiment, the communicated data is used to synchronize the mechatronic devices. In one embodiment, one or more of the mechatronic devices is configured to receive executable instructions for controlling an actuator via a communications interface.

## Description

### RELATED APPLICATIONS

This application claims the benefit of, and incorporates by reference in their entirety, U.S. Provisional Application No. 60/653,717, filed February 16, 2005 and U.S. Provisional Application No. 60/679,953, filed May 10, 2005.

### BACKGROUND OF THE INVENTION

### Field of the Invention

The present invention relates to prosthetic and orthotic limbs in general and, in addition, a system and method of configuring and synchronizing the adaptive control systems of prosthetic and orthotic devices on a patient.

### Description of the Related Art

Prosthetic and orthotic devices, such as are attached to a human limb, have benefited from advances in electronics. Electronically controlled prosthetic or orthotic devices, which may be generally referred to as "mechatronic" devices, for example, prosthetic knees, can provide safer and more natural movement to patients who are equipped with such systems. However, advances in electronics appear to have outpaced the advances in control systems. Thus, control systems for prosthetic systems can benefit from intelligent architectures.

Further, the proliferation of electronic control systems for prosthetic and orthotic systems has created a need for systems and methods of synchronizing multiple devices which are worn by a single patient, e.g., a prosthetic knee and a prosthetic ankle. Operating in isolation from each other, multiple control systems may fail to provide the patient with stable, coordinated movement. In addition, independent configuration of multiple prosthetic devices can be inconvenient. Thus, it is desirable to have systems and methods of configuration, communication, and synchronization between such control systems. Further, it is desirable to have systems and methods of adding, replacing, or augmenting portions of the software in such control systems.

### Summary of the Certain Embodiments

After considering this discussion, and particularly after reading the section entitled "Detailed Description of Certain Embodiments" one will understand how the features of this invention provide advantages that include providing a prosthetic or orthotic control system that provides more natural and comfortable movement to its users and enabling a more convenient and intuitive configuration, addition, replacement, or augmentation of control system software.

One embodiment is a system for controlling motion of a human limb. The system may include a plurality of mechatronic devices. Each of the plurality of mechatronic devices is in communication with at least one other of the plurality of mechatronic devices. At least one of the mechatronic devices controls an actuator. In one such embodiment, at least one of the plurality of mechatronic devices is configured to generate a control state for at least one other of the plurality of mechatronic devices based on the communicated data. In one embodiment, the communicated data is used to synchronize the mechatronic devices. In one embodiment, each of the mechatronic devices comprises an artificial joint. In one embodiment, at least one of the plurality of mechatronic devices comprises a prosthetic knee and at least one of the mechatronic devices comprises a prosthetic ankle.

Another embodiment is a mechatronic device for controlling motion of a human limb in cooperation with at least one other mechatronic device. The mechatronic device includes a communication interface configured to communicate data with the at least one other mechatronic device, a sensor configured to obtain a value indicative of at least one motion parameter of the limb; an actuator configured to affect at least one motion parameter of the mechatronic device, and a processor configured to activate the actuator based on the received communicated data and the at least one motion parameter value. In one embodiment, the communicated data may include the parameter value obtained from the sensor. In another embodiment, the communicated data may include state machine data received from tha other mechatronic devices: In yet another embodiment, the communicated data may include configuration data received from the other mechatronic devices.

Another embodiment is a mechatronic device for controlling motion of a human limb in cooperation with at least one other mechatronic device. The mechatronic device includes a communication interface configured to communicate data with the at least one other mechatronic device; and a processor configured to generate a control state of the at least one other mechatronic device. The processor is further configured to communicate data associated with the control state through the communication interface. The mechatronic device further includes an actuator controlled by the processor so as to effectuate movement of the human limb. In another embodiment, the communicated data may include software that when executed by the processor is configured to affect the selection of the control state. In one embodiment, the communicated data includes data obtained by the at least one sensor of the other mechatronic device. In one embodiment, the communicated data includes configuration data obtained by the at least one sensor of the other mechatronic device. In one embodiment, the processor is further configured to determine at least one actuator control command based on the control state, and wherein the communicated data includes the at least one actuator control command.

Another embodiment is a method of synchronizing a first mechatronic device with a second mechatronic device. The method includes communicating data from the second mechatronic device to the first mechatronic device. The method further includes generating a control state in response to the received data. The method further includes controlling an actuator on the second mechatronic device based at least in part on the control state. In one embodiment, the method further includes generating a command to control an actuator of the second mechatronic device in response to the control state. In one embodiment, the method further includes generating a command to control an actuator of the first mechatronic device in response to the communicated data. In one embodiment, the received data includes sensor data received from the second mechatronic device. In another embodiment, the received data includes at least a portion of information indicative of the control state. In yet another embodiment, the received data includes computer software and the control state is performed at least partly by executing the computer software.

Another embodiment is a system for controlling motion of a device associated with a limb. The system includes a mechatronic device. The system further includes a sensor associated with a human limb which provides motion parameter data to the mechatronic device. The mechatronic device uses the motion parameter data for synchronization. In one embodiment, the sensor receives signals from the human nervous system. In one embodiment, the sensor receives signals from a sensor associated with a sound limb. In one embodiment, the motion parameter data is used for synchronization with another mechatronic device. In one such embodiment, the other mechatronic device provides motion parameter data to the mechatronic device.

One embodiment is a method of synchronizing a computing device with a a device associated with a limb. The method includes communicating data between the mechatronic system and the computing device, storing the data on the computing device, generating a control state on the mechatronic system in response to the data, and controlling an actuator on the second mechatronic system based at least in part on the control state.

Another embodiment is a mechatronic system attached to a human body. The device includes a sensor configured to provide data indicative of movement of the human body. An actuator is configured to control movement of at least a portion of the human body. A processor is configured to execute instructions configured to control the actuator based on the sensor data. A communication interface is configured to communicate data with a data source. The processor is further configured to receive at least a portion of the instructions from the data source. In one embodiment, the mechatronic system may include a separation of the processing, sensing, actuation, and communications in two or more mechatronic devices.

### BRIEF DESCRIPTION OF THE DRAWINGS

Figure 1 is a block diagram that illustrates one embodiment of a system including a number of mechatronic devices.

Figure 2 is a block diagram illustrating in more detail one embodiment of a mechatronic device in communication with additional devices in one embodiment of the system of Figure 1.

Figure 3 illustrates a user interface of one embodiment of an instrumentation program for use with a mechatronic device.

Figure 4A is a schematic block diagram of an exemplary embodiment of the system of Figure 1 that includes a prosthetic knee and a prosthetic ankle.

Figure 4B is a schematic block diagram of an exemplary embodiment of the system of Figure 1 that includes a prosthetic knee and a prosthetic foot.

Figure 4C is a schematic block diagram of another exemplary embodiment of the system of Figure 1 that includes a prosthetic knee, a prosthetic foot, and a master device.

Figure 4D is a schematic, block diagram of another exemplary embodiment of the system of Figure 1 that includes a prosthetic knee and a prosthetic foot in which the prosthetic foot includes one or more state machines for controlling both devices.

Figure 5 is a block diagram illustrating one embodiment of a system including mechatronic devices in communication with personal and network computing devices.

Figure 6 is a flowchart illustrating one embodiment of a method of synchronizing configuration or calibration data of the mechatronic device with the network computing device.

Figure 7 is a flowchart illustrating one embodiment of a method of replacing or augmenting software on the mechatronic device.

### DETAILED DESCRIPTION OF CERTAIN EMBODIMENTS

The following detailed description is directed to certain specific embodiments of the invention. However, the invention can be embodied in a multitude of different ways. In this description, reference is made to the drawings wherein like parts are designated with like numerals throughout.

The terms "prosthetic" and "prosthesis" as used herein are broad terms and are used in their ordinary sense and refer to, without limitation, any system, device or apparatus that may be used as an artificial substitute or support for a body part.

The term "orthotic" and "orthosis" as used herein are broad terms and are used in their ordinary sense and refer to, without limitation, any system, device or apparatus that may be used to support, align, prevent, protect, correct deformities of, immobilize, or improve the function of parts of the body, such as joints and/or limbs.

A device associated with, a limb is any device that may be used to assist the limb in some function. For instance, a prosthetic device is a device associated with a limb. A prosthetic device may replace a portion of or the entire limb. Alternatively, an orthotic device is a device associated with a limb. An orthotic device, for instance, supports or aligns the limb. Additionally, other devices, such as articles of clothing or sporting goods equipment, may be devices associated with a limb. For instance, a shoe is a device associated with a limb because it assists the user of the shoe to use the foot, for example, to walk or run. Similarly, a ski boot is a device associated with a limb because it assists the user of the ski boot to use the foot, for example, to ski.

The term "mechatronic" as used herein is a broad term and is used in its ordinary sense and refer to, without limitation, any system, device, or apparatus that includes an electronically controlled device associated with a limb, including a prosthetic or orthotic device. Such devices may include one or more of a sensor, an actuator, or processor.

The term "bionic" as used herein is a broad term and is used in its ordinary sense and refer to, without limitation, any system, device, or apparatus that includes an electronically controlled device integrated to replace or enhance anatomical structures or physiological processes. Bionic may also include electronic or mechanical smart structures or systems integrated to replace or enhance anatomical structures or physiological processes. For example, a bionic may include a mechatronic device such as prosthetic or orthotic.

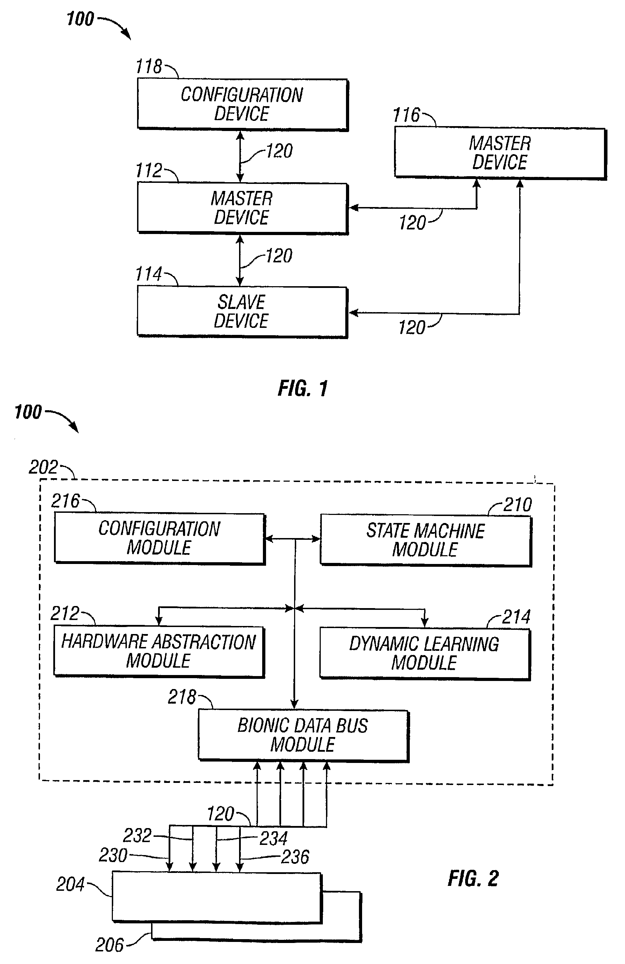

Users of prosthetic or orthotic devices often may need more than one device. For example, a trans-femoral amputee may require a combination of a mechatronic knee and a mechatronic ankle or foot. Typically, more natural movement may be achieved when these devices are coordinated. Where two or more of these devices are electronically controlled devices, improved coordination, e.g., from a more natural motion, can be achieved by electronic interface and coordination between the devices. Figure 1 is a block diagram that illustrates one embodiment of a system 100 which includes multiple mechatronic devices. In one embodiment, a particular mechatronic device includes one or more sensors, a controller, and one or more actuators. However, it is to be recognized that in other embodiments a particular mechatronic device may include, for example, only sensors, sensors and a controller, one or more actuators, actuators and a controller, or only a controller. In one embodiment, the system may include a master device 112. In one embodiment, the master device 112 directs control of the entire system 100. In one embodiment, the master device 112 is a mechatronic device that has a control system which incorporates a state machine. The master device 112 may fully or partially control a slave device 114. Information on state changes or direct actuation commands may be sent to components of the system 100, such as the slave device 114. Embodiments of each of the devices in the system 100 may include prosthetic knees, prosthetic ankles, or other electronically controlled prosthetic or orthotic devices. For example, an orthotic device such as a brace may include a sensor for measuring knee motion.

In one embodiment, the slave device 114 may only include a portion of the software or hardware needed to control the slave device 114. The slave device 114 may thus be wholly or partially dependent on receiving state information and commands from the master device 112. In one embodiment, the slave device 114 may receive sensor data from the master device 112, or another slave device 114. The slave device 114 may also send sensor data to other devices 112, 114, 116, or 118. In one such embodiment, the slave device 114 includes one or more sensors but does not include an actuator.

The system 100 may include an observation device 116 that is configured to monitor or control one or more of the other devices in the system 100. In one embodiment, the observation device includes a wristwatch, or arm mounted device, that provides status or other information regarding the operation of devices in the system 100. In one embodiment, the status information is updated in real-time. In another embodiment, the observation device 116 may have controls configured to affect the operation of the system 100. In one such embodiment, the observation device 116 includes only a controller that is configured to receive sensor data and/or send control data to other mechatronic devices in the system 100. For example, in one embodiment, the master device 112 may be a prosthetic knee and the observation device 116 may be used for activation or to provide hints as to different use modes, e.g., walking, bicycling, etc.

The system 100 may also include a configuration device 118 that is adapted to control one or more of the other devices in the system. In one embodiment, the configuration device 118 is in direct communication with the master device 112. The master device 112 coordinates communication of configuration data with other devices, e.g., the slave device 114 or the observation device 116. In other embodiments, the configuration device 118 may be in direct communication with all or any subset of the devices 112, 114, 116.

Each of the devices 112, 114, 116, and 118 of the system 110 may communicate using a bionic data bus (BDB) 120. The BDB 120 may comprise any data communications physical layer, including those known in the art. For example, the BDB 120 may include one or more of the following communications layers: a remote modem, Ethernet (IEEE 802.3), Token Ring (IEEE 802.5), Fiber Distributed Datalink Interface (FDDI) Asynchronous Transfer Mode (ATM), Wireless Ethernet (IEEE 802.11), Bluetooth (IEEE 802.15.1), or infrared interfaces including IRDA. The BDB bus may also include a peripheral interface bus including Universal Serial Bus (USB), IEEE 1394, Peripheral Component Interconnect (PCI), or other peripheral buses such as those known in the art. In addition, the BDB 120 may include networks such as an Intranet, a Local Area Networks (LAN), a Wide Area Network (WAN), or the Internet. The BDB 120 may include additional protocols such as internet protocol (IP) or transmission control protocol (TCP).

It will be recognized that while, in one embodiment, a mechatronic device may operate as one of the devices 112, 114, 116, and 118, in other embodiments of the system 100, a particular mechatronic device may be configured to operate in different modes or roles as one or more of the devices 112, 114, 116, and 118. In one embodiment, the particular mechatronic device may be configured to automatically act as a particular type of device based on data exchange with other devices in the system 100. For example, one embodiment of the system 100 may include a prosthetic knee, a prosthetic ankle, and a wrist-attached monitor. Embodiments of prosthetic knees may include those illustrated in U.S. Patent No. 6,610,101, filed March 29, 2001, and issued on August 26, 2003; U.S. Patent Application No. 11/123,870, filed May 6, 2005; and U.S. Patent Publication No. 2005-0283257, filed on March 9, 2005; each of which is incorporated by reference in its entirety. Embodiments of prosthetic ankles may include those illustrated in U.S. Patent Publication No. US 2005-0197717, filed February 11, 2005, and which is incorporated by reference in its entirety.

After exchanging identifying data over the BDB 120, the knee may configure itself to operate as the master device 112, the ankle may configure itself to operate as a slave device 114, and the monitor to configure itself as an observation device 116. In another embodiment of the system 100 that includes only the ankle and the wrist monitor, the ankle may configure itself as the master device 112 and the monitor as the observation device 116.

In one embodiment, devices may include a configuration database. The database may contain data relating configurations of the system 100 with the role of the device. For example, the ankle device may include data indicating that the ankle should configure itself as the slave device 114 when the system 100 includes a knee prosthetic, but should configure itself as the master device 112 in other configurations.

It will be further recognized that in some embodiments, the system 100 may include one or more of each of the slave device 114, observation device 116, and configuration device 118. Further, in some embodiments, multiple master devices may be configured such that the devices each control groups of prosthetics, e.g., one master device 112 for a group of arm based mechatronic devices and a second master device 112 for a group of leg based mechatronic devices. In such an embodiment, the observation device 116 may display information related to some of the master and slave devices 112 and 114. In another embodiment, each observation device 116 may display information related only to a single master or slave device 112 or 114.

The master devices 112 may communicate over the BDB 110 to share data or otherwise coordinate operation of the system 100. In one such embodiment, each of, e.g., arm and leg mechatronic devices may operate as the master device 112 with respect to a group of devices. For instance, the knee may operate as the master device 112 with respect to an ankle prosthesis and a shoulder mechatronic device may act as a master device 112 to an elbow slave device 114. Continuing with this exemplary embodiment, with respect to knee master device 112, the ankle may operate as a slave device 114.

It will be recognized that the devices 112, 114, 116, 118 as described herein refer to roles or functional descriptions of one mode of operation of a mechatronic device. In some embodiments, a mechatronic device may be a hybrid device, e.g., one that acts as a slave device 112 under the influence or direction by another master device 112, but which also maintains a distinct state machine. Further, other embodiments may include mechatronic devices that operate as combinations of any of the devices described herein.

Figure 2 is a block diagram illustrating in more detail one embodiment of a mechatronic device 202 in communication with additional devices 204 and 206 in one embodiment of then system 100 via the BDB 120. The device 202 may include a processor and memory configured to execute software for controlling the operation of the device.

In one embodiment, the software includes a state machine module 210, a hardware abstraction module 212, a dynamic learning module 214, a configuration module 216, and a BDB module 218. It will be recognized that each of the modules 210, 212, 214, 216, and 218 may include various sub-routines, procedures, definitional statements and macros. Each of the modules may be separately compiled and linked into a single executable program. The description of each of the modules is used for convenience to describe the functionality of one embodiment of a system. Thus, the processes that are performed by each of the modules may be redistributed to one of the other modules, combined together in a single module, or made available in, for example, a shareable dynamic link library. In some embodiments, the modules may be executed concurrently or in parallel as distinct threads or processes. The modules may be produced using any suitable computer language or environment, including general-purpose languages such as C, C++, Java, or FORTRAN.

Each of the modules 210, 212, 214, 216, and 218 may communicate via any suitable method such as are known in the art. In one embodiment, the modules may communicate using shared data structures such as are described in U.S. Patent Publication No. 2005-0283257, filed on March 9, 20054, which was previously incorporated herein. In one embodiment, the shared data structure may include portions that are available for access through the bionic data bus module 218 to other devices 204 and 206 in the system 100. In such an embodiment, portions of the data in the shared structure may be communicated on the BDB 120.

In one embodiment, the observation device 116 may be a personal or server computer system configured to perform diagnostic functions of other devices in the system 100. In one embodiment, the observation device 116 may be configured to receive and update the contents of shared data structures, such as described above, through the bionic data bus module 218.

The state machine module 210 typically includes high level, application or device specific instructions. The state machine module 210 may be generally described as having the intelligence of the device. The state machine module 210 of a particular embodiment of a mechatronic device may be configured to operate as the master device 112, the slave device 114, the observation device 116, or the configuration device 118 in various embodiments of the system 100. An embodiment of the state machine module 210 may be configured so as to be loaded into different mechatronic devices, e.g., different knee hardware, without modification by using the hardware abstraction module 212 to interface with specific hardware on a particular mechatronic device. One exemplary embodiment of a state machine module 210 is described in U.S. Patent No. 6,610,101, filed March 29, 2001, and issued on August 26, 2003, incorporated above.

In one embodiment, portions of the state machine module 210 may be replaced or augmented to provide customized, e.g., activity based, control of the mechatronic system 100. For example, software for a specific activity, e.g., bicycling or jogging, may be installed into the state machine module 210 to improve or customize the functionality of the mechatronic device, e.g., a prosthetic knee, for the specific activity. In one embodiment, the customized control software is installed via download. In one embodiment, the downloaded data may be received from the configuration device 118. In another embodiment, the master device 112 may include a network interface over which the customized control software may be received from any other networked computing device. The network interface may comprise a wireless network, e.g., a mobile telephone network, or any other suitable computer network, such as those discussed above in connection with the BDB 120.

The hardware abstraction module 212 typically includes low level, hardware specific code that provides a standardized interface to the hardware by other software modules. The hardware abstraction layer 212 may abstract hardware such as sensors and actuators. The hardware abstraction module 212 thus allows other software, such as the state machine module 210 to be reused with different sensors so long as the sensors each provide data that the hardware abstraction module 212 can represent in a standardized form. For example, a particular sensor may provide data via setting the value of a hardware register. Another sensor for producing equivalent data may signal the processor via an interrupt when the data is updated. The hardware abstraction layer 212 can be configured to read either sensor and provide the data using a uniform interface so that other software layers do not need to be modified if the particular sensor changes. This may be particularly desirable in the system 100 having multiple mechatronic devices 202, 204, 206. For example, an ankle mechatronic device 202 may be configured to receive a sensor value, e.g., a knee angle, from different types and models of prosthetic knees 204. Continuing this example, the hardware abstraction layer 212 of the ankle device 202 may provide, in one embodiment, a knee angle that is updated every 5 milliseconds regardless of whether the sensor is configured to be polled by the processor to receive updates or whether the sensor signals the processor via, e.g., an interrupt channel. The hardware abstraction layer 212 may also be configured to provide the knee angle value that is upsampled or downsampled to a consistent, accurate value regardless of the sensor resolution. For example, the knee angle value may be represented with a value having a resolution of 8 bits, 10 bits or higher. Moreover, the interface to the data may be the same regardless of whether the data is coming from the same mechatronic device 202 or other mechatronic devices 204, 206.

It is to be recognized that some embodiments include mechatronic devices in which the hardware abstraction layer 212 is configured to communicate with a patient's nervous or muscular system. For example, the actuator may include a muscle. In one embodiment, a sensor includes a nerve of the patient's body.

The dynamic learning module 214 may include a dynamic learning matrix that updates runtime parameters such as may be used by the state machine module 212. In one embodiment, the learning module 214 may adapt runtime parameters to the current pace of movement, particular activity, terrain, etc. One exemplary embodiment of a learning module 214 is described in U.S. Patent No. 6,610,101, filed March 29, 2001, and issued on August 26, 2003, incorporated above.

The configuration module 216 may be configured to store and maintain control parameters. The parameters may be subsequently automatically adjusted by the learning module 214 or through the configuration device 118. In one embodiment, the data maintained by the configuration module 216 is substantially static. The configuration module 216 may be configured to communicate with the BDB 120 to the configuration device 118 to send and receive parameter data. The configuration module 216 may provide a standard interface over the BDB 120 to the configuration device 118. In one embodiment, the configuration module 216, e.g., of the slave device 114 is configured to receive parameters through other devices such as the master device 112. Thus, the components of the system 100 may be configured together through the configuration device 118 in communication with the master device 112, which further communicates parameters to other devices such as devices 204 and 206 in the system 100.

In one embodiment, the abstraction module 212 controls one or more actuators in a mechatronic system 100. An actuator may include, for example, a In one embodiment, this comprises applying damping through an actuator in, e.g., a prosthetic knee. In one embodiment, at least a portion of the abstraction module 212 executes at a frequency that is different from the execution rate of the state machine or learning modules 210 and 214. For example, in one embodiment the low level abstraction module 212 executes with a period of 1 millisecond (ms) while the higher level code of the state machine executes with a period of 5 ms.

The bionic data bus (BDB) module 218 is configured to provide data communications between devices in the system 100 over the BDB 120. One embodiment of the BDB module 218 includes a software interface that abstracts or standardizes an interface to the other modules 210, 212, 214, and 216 for communicating over the BDB 120 regardless of the particular embodiment of the BDB 120, e.g., regardless of whether the BDB includes a network or a peripheral bus such as USB.

The BDB module 218 may provide a layered interface to the BDB 120. In one embodiment, the layers may correspond to one or more physical channels provided by the BDB 120. In other embodiments, the layers may correspond to logical channels over the BDB 120. In one embodiment, the channels provided by the BDB module 218 includes a state channel 230, a parameter channel 232, a sensor channel 234, and an actuation channel 236.

The state channel 230 may be configured to communicate high frequency, low volume state machine data between mechatronic devices. In one embodiment, this data may include data related to the gait cycle of a prosthetic knee. The data may include state data or state change data. For example, in a prosthetic knee, the state change may indicate a change in a gait cycle.

The parameter channel 232 may be configured to communicate data at intermediate frequencies and volumes to communicate parameter settings between devices, e.g., between the configuration device 118 and the master device 112. The parameter channel 232 may data may include configuration parameters such as are described in U.S. Patent Publication No. 2005-0283257, filed on March 9, 2005, which was previously incorporated herein.

The sensor channel 234 may be configured to communicate high frequency, low volume sensor data. Sensor data from one device in the system 100 may thus be shared for use by other devices. This allows for placement of sensors in locations that are not physically located in or adjacent to a particular mechatronic device but which are physically located within or adjacent to another device in the system 100. Moreover, certain sensors may thus be shared to reduce overall cost of the system 100. Sensors may include force sensors, battery voltage sensors, or any other sensors as may be incorporated or attached to any mechatronic device.

Another channel may include the actuation channel 236. The actuation channel 236 communicates low volume, high frequency data that includes actuator control signals. In one embodiment, the master device 112 may send actuator control signals over the actuation channel 236 to control an actuator on the slave device 114. The data may include data such as position, force, direction, and velocity.

In addition to communicating with other mechatronic devices, other electronic devices, e.g., a remote server computer (not shown), may communicate with the mechatronic device via the BDB 120. In one embodiment, the remote server may carry out maintenance activities such as diagnosing faults in the mechatronic device. The device 202 may communicate sensor data, state change data, or other data generated on the device 202, or devices 204, 206 attached to the device 202 via the BDB 120.

In one embodiment, a common naming convention is used to identify the data communicated on the channels. In one embodiment, the data is formatted as structured data using the naming convention, such as in extendible markup language (XML). In one embodiment, the naming convention is based on using terminology analogous to anatomical equivalents. For example, in one embodiment, the naming convention includes terminology from the human muscular system for actuator signals and from the human nervous system for sensor signals.

In addition to communicating with other mechatronic devices, other electronic devices, e.g., a remote server computer (not shown), may communicate with the mechatronic device via the BDB 120. In one embodiment, the remote server may carry out maintenance activities such as diagnosing faults in the mechatronic device. The device 202 may communicate sensor data, state change data, or other data generated on the device 202, or devices 204, 206 attached to the device 202 via the BDB 120.

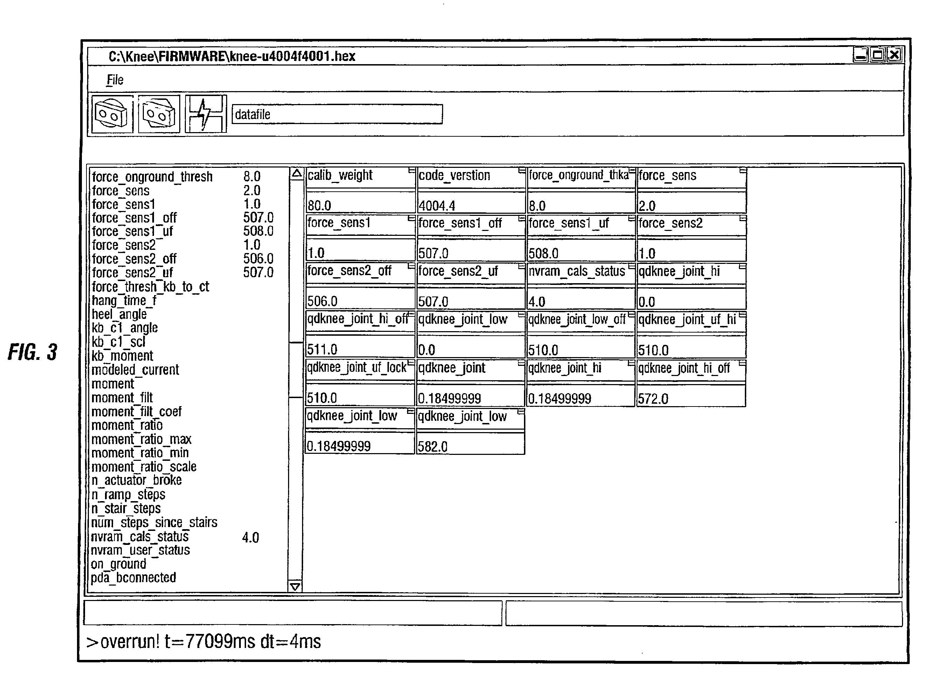

In one embodiment, the remote computer includes instrumentation software for maintenance or development of the mechatronic device 202. Figure 3 illustrates a user interface of one embodiment of the instrumentation program for use with a prosthetic knee. The left column displays the names of memory locations, registers, or other data that may be monitored on the mechatronic device 202. In the depicted embodiment, selecting the name of a monitored item causes the value to be displayed. In one embodiment, the displayed value is continuously and automatically updated when new data is received from the device 202. In one embodiment, the values of the monitored items may be recorded to a file for later analysis. This analysis may include graphical plotting of the data. In one embodiment, the instrumentation program may also send commands to the device 202, such as to erase data, reset the device 202, and update the software or firmware on the device 202. In one embodiment, the values of these items may be modified by a user of the instrumentation program. In one embodiment, the instrumentation program may be configured to restrict the values of the updated items to be set within a predetermined range.

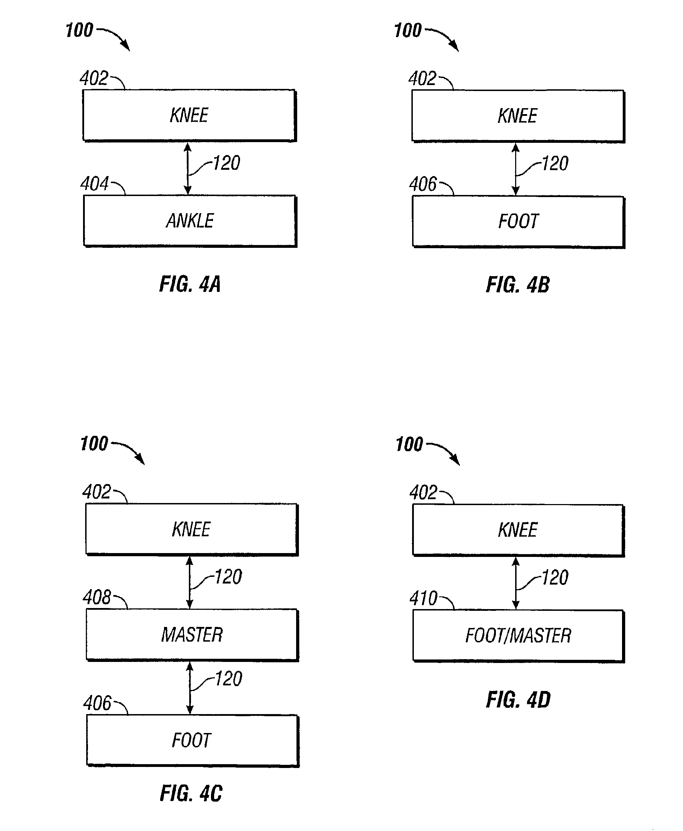

Figure 4A is a schematic block diagram of an exemplary embodiment of the system 100 that includes a prosthetic knee 402 and a prosthetic ankle 404. When the system 100 includes an electronically controlled ankle 404 and an electronically controlled knee 402 there is a risk of instability if the two "intelligent" components do not share information or otherwise work in a synchronized manner. The knee 402 may include 3 main sensors, an angle sensor, posterior force sensor (PF) and anterior force sensor (AF). From the signals of PF and AF sensors, the knee 402 can calculate the moment in a pylon. The knee 402 can represent the moment as information as to how much the toe is being loaded and how much the heel is being loaded. From the calculation on the values from PF and AF sensors, the knee 402 is also able to tell if the foot is placed on the ground and with how much force. The force signals together with the angle sensor are evaluated by an algorithm in the state machine module to define the state of the knee 402 in a high level loop cycling, in one embodiment, every 5 ms. If the signals are incorrect or misinterpreted, the knee 402 cannot change states or function correctly.

Since the values from the force sensors (bending moment in the knee frame) are translated into toe- and heel load values, the alignment of the foot and especially the angle of the ankle 404 should be determined. During setup, certain ranges and threshold values may be set for the knee 402. If the alignment is changed considerably after the initial setup, the knee 402 can misinterpret the information from the force sensors. The functionality of an electronically adjusted ankle 404 typically causes just such a change in alignment.

If the ankle 404 can send information on the angle value to the knee with a sufficiently high frequency, the knee can compensate for the "error" in force signals from the sensors and the whole system 100 can operate in a more stable way as compared to a non-synchronized system.

The electronic ankle 404 may also be designed to also fit below-the-knee amputees. In such a mode of use, the ankle 404 does not need the extra information from a "colleague" component. The extra information that the knee 402 is able to communicate may however simplify the design of the ankle for use by above-the-knee amputees.

In addition, the use of data from the knee 402 by the ankle 404 can provide additional functionality to the system 100. For example, the angle value of the ankle 402 can be made accessible to the knee 404 through the sensor channel 232 of the BDB 120. Also if the ankle is offset by some degree (for use with high heels, for example), the knee 402 may use the information to further compensate for the force sensor measurements. The offset value can be communicated over the parameter channel 232.

In one embodiment, the ankle may include a prosthetic or orthotic foot, similar to embodiments disclosed in U.S. Patent Application No. 11/346,600, filed on February 2, 2006, titled "SENSING SYSTEMS AND METHODS FOR MONITORING GAIT DYNAMICS," and incorporated by reference in its entirety, that is configured to make and provide toe load and heal load measurements over the BDB 120. In another embodiment, the ankle may include a prosthetic or orthotic foot, similar to embodiments disclosed in U.S. Patent Application No. 10/742,455, filed on December 18, 2003, titled "Prosthetic foot with rocker member," and incorporated by reference in its entirety, that is configured to make and provide an angle measurement over the BDB 120.

Figure 4B is a schematic block diagram of an exemplary embodiment of the system of Figure 1 that includes a prosthetic knee 402 and a prosthetic foot 406. In one embodiment, the knee 402 and the ankle 404 each include a data communications or network interface such as an RS-232 port that are in communication with each other to define the BDB 120. In another embodiment, the BDB 120 may be implemented via RS-485 ports on each of the devices 402 and 406. In one embodiment, the prosthetic foot 406 includes a joint that allows the foot to adjust to different grades of slopes. As a result, the response from the foot 406 will differ from prosthetic feet with a fixed ankle. In one embodiment, the knee 402 is controlled based on force measurements that are translated into bending moments. From the moment values, the knee 402 manages state changes and adjusts the resistance of the knee based on whether the knee 402 is on level ground, on different grades of slopes, or on stairs.

In one embodiment, the knee 402 may detect that the user is walking on a sloped surface based on changes in force and moment. Due to bending of the jointed foot 406, the foot 406 may adjust to a slope so that the knee 402 does not receive force measurements that are consistent with walking on the slope. Thus, the knee 402 may act as if the user is walking on level ground when the user is actually descending a ramp. In one embodiment, the foot 406 may communicate its joint angle to the knee 402 when the angle has changed. In other embodiments, the foot 406 may communicate the angle to the knee at a predetermined rate or when the angle changes by a threshold amount. In one embodiment, the knee 402 may request the data from the foot 406 either at intervals or response to particular events such as state changes. The knee 402 may then use the angle value to correct the moment calculations (e.g., through a proportional calculation as a function of the angle). In one embodiment, the data communicated from the foot 406 to the knee 402 may include state machine data. The state machine data may be used by the control system of the knee 402 to coordinate movement with the foot 406 and to better identify the proper control response based on the additional information from the foot 406, e.g., correcting force sensor readings when the joint of the foot 406 is bent.

Data may be communicated between the foot 406 and the knee 402 using any suitable protocol such as discussed above with reference to the BDB in Figure 1. For example, in one embodiment, sensor and control data may be communicated as a string of characters over the RS-232 link. In one embodiment, in each program cycle of the knee 402, the knee reads the serial port, parses the string and filters out the angle value. The angle value is then translated into a correction value for a slope detection routine.

In another embodiment, data may be communicated over the RS-232 layer by a suitable link layer protocol such,as the High Level Link Control (HDLC) protocol. In other embodiments, suitable higher level protocols may be used. In one embodiment, the two RS-232 ports may be connected via simple wire interface.

In one embodiment, the knee 402 may operate as the master device 112 that receives sensor data from the foot 406 and use that data to generate control signals that are communicated back to the foot 406. In such an embodiment, the additional sensor data from the foot 406 may be used to provide control that is more robust and enable the knee 402 to be better able to anticipate or otherwise manage state changes. Moreover, the additional sensor data of the knee can be used to extend or improve the control of the foot 406. For example, the load sensors of the knee 402 may be able to detect a rapid toe off signal that can indicate initial steps onto stairs. The control system of the foot 406 may be configured to use this data to anticipate and better detect state changes such as stair ascent or descent.

In one embodiment, the foot 406 and the knee 402 may also be configured to share a power source. In such an embodiment, the master device 112, e.g., the knee, may coordinate power management for both devices. In one embodiment, the foot 406 and knee 402 may be designed specifically to operate together. However, in other embodiments, any knee 402 and foot 406 that include compatible mechanical and communication interfaces may form the system 100.

Figure 4C is a schematic block diagram of another exemplary embodiment of the system of Figure 1 that includes a prosthetic knee 402, a prosthetic foot 406, and a master device 408 operating as a master device 112. The master device 408 may include any electronic device configured to receive sensor data from each of the knee 402 and the foot 406 and provide control signals to the knee 402 and the foot 406 based on that sensor data.

Figure 4D is a schematic block diagram of another exemplary embodiment of the system of Figure 1 that includes a prosthetic knee 402 and a prosthetic foot 406 in which the prosthetic foot 406 operates as the master device 112. In such an embodiment, the controller of the foot 406 may include one or more state machines for controlling both devices.

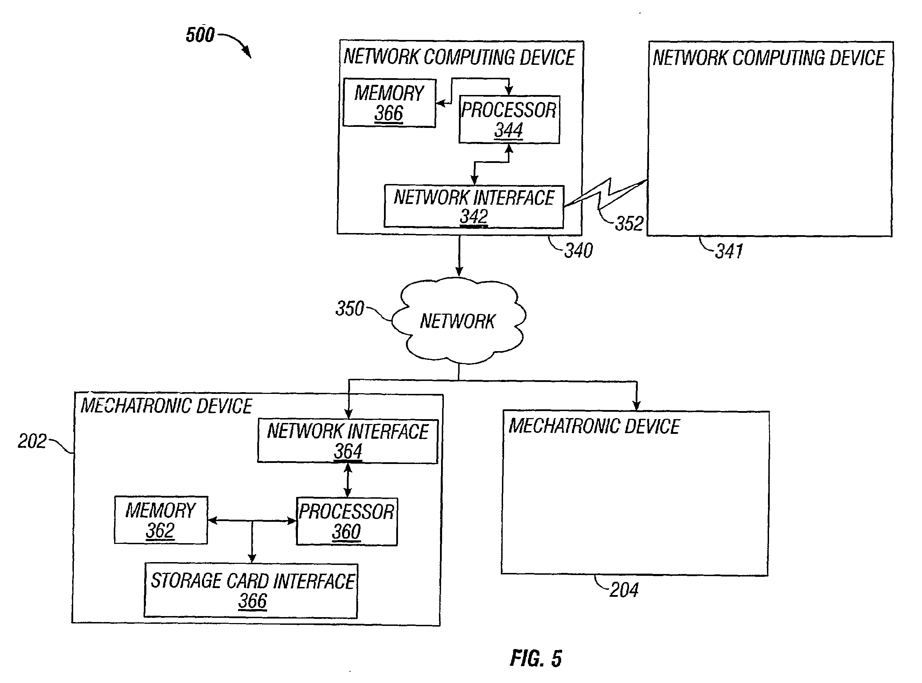

Figure 5 is a block diagram that depicts one embodiment of a system 500 for communicating with a pair of mechatronic devices 202 and 204. In the depicted embodiment, the system 500 includes a single network computing device 340 in communication with the mechatronic devices 202 and 204 via a data communications network 350. Other embodiments include only a single mechatronic device 202, or more than two mechatronic devices. In one embodiment, the system 500 includes additional network computing devices 341 that are also in communication with the network computing device 340 via a network 352. In one embodiment, the mechatronic devices 202 and 204 are configured to communicate with the network computing device 340 to send and receive configuration and calibration data. In one embodiment, the mechatronic devices 202 and 204 are configured to communicate with the network computing device 340 to receive executable instructions to augment or replace portions, or all, of one or more of the state machine module 210, the hardware abstraction module 212, the dynamic learning module 214, a configuration module 216, the BDB module 218, or any other suitable software module of the mechatronic device 202.

In one embodiment, the network computing device 340 includes a network interface 342 in communication with a processor 344 and a memory 346. The network computing device 340 may include a server computer, a personal computer, or a mobile computer such as a laptop computer. In one embodiment, the network computing device 340 includes a personal digital assistant. In another embodiment, the network computing device 340 includes a mobile telephone.

The network interface 342 provides network connectivity to one or more computing devices, including the mechatronic devices 202 and 204, via the networks 350 and 352. In one embodiment, the network interface 344 to the networks 350 and 352 includes one or more of, for example, a remote modem, Ethernet (IEEE 802.3), Token Ring (IEEE 802.5), Fiber Distributed Datalink Interface (FDDI) Asynchronous Transfer Mode (ATM), Wireless Ethernet (IEEE 802.11), Bluetooth (IEEE 802.15.1), or infrared interfaces including IRDA. The network 350 may include networks such as the Internet, an intranet, Local Area Networks (LAN) or Wide Area Networks (WAN). As used herein, the networks 350 and 352 may include network variations such as the public Internet, a private network within the Internet, a secure network within the Internet, a private network, a public network, a value-added network, an intranet, and the like. In one embodiment, the network 350 includes the network 352.

The processor 344 may be any suitable general purpose single- or multi-chip microprocessor such as an ARM, Pentium^{®}, Pentium II^{®}, Pentium III^{®}, Pentium IV^{®}, Pentium^{®} Pro, an 8051, a MIPS^{®}, a Power PC^{®}, an ALPHA^{®}, or any other suitable processor. In addition, the processor 344 may comprise any suitable special purpose microprocessor such as a digital signal processor or a programmable gate array.

The memory 346 may include volatile components, such as, for example, DRAM or SRAM. The memory 346 may also include non-volatile components, such as, for example, memory or disk based storage. In one embodiment, the network computing device 340 includes a server and the memory 346 includes disk base storage. In one embodiment, the disk based storage includes a file server.

In one embodiment, the mechatronic device 202 includes a storage card interface 366 to a removably connected memory. The storage card interface 366 may include an interface to a removable storage card that includes semiconductor storage (chips), for example, Random Access Memory (RAM) or various forms of Read Only Memory (ROM), that are removablely connected to the processor 344. Removably connected memory may include memory on any standardized or proprietary device such as a memory card, a secure digital memory card, a memory stick, or any other suitable removable memory device. In one embodiment, the storage card interface 400 is configured to interface the processor solid state persistent memory such as FLASH memory or magnetoresistance RAM (MRAM). In one embodiment, the memory 104 includes a disk drive, e.g., a magnetic, optical, or magneto-optical drive.

In one embodiment, each of the mechatronic devices 202 and 204, includes a processor 360 connected to a memory 362 and a network interface 364. The processor 360 may include any suitable processor including those discussed above with respect to the processor 344. The memory 362 may include any suitable memory such as discussed above with respect to the memory 346. The network interface 364 places the processor 360 in communication with the network 350. The network interface 364 may include any suitable network interface, including those discussed above with respect to the network interface 342.

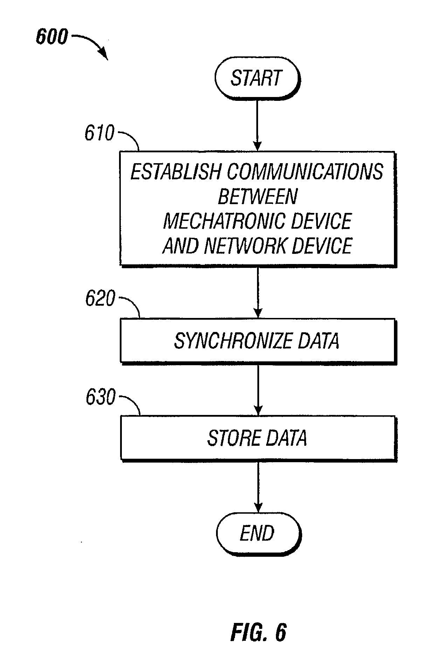

Figure 6 is a flowchart illustrating one embodiment of a method 600 of synchronizing configuration or calibration data of the mechatronic device with the network computing device 340 of Figure 5. Configuration data may include data that is entered by a prosthetist, determined based on predetermined parameters, such as the height of a user of the mechatronic device, selected based on experience or preferences of the user of the mechatronic device 202, or selected by a designer or manufacturer of the mechatronic device, that affects the control system of the mechatronic device 202. Calibration data may include data that is determined by the control system of the mechatronic during operation of the mechatronic device 202. Such data may also be generally referred to as control data. The method 600 begins at a block 610 in which the mechatronic device 202 establishes communications with the network computing device 340. Entry or examination of such data could be made through a screen display such as the one shown in Figure 3.

Next at a block 620, the mechatronic device 202 synchronizes one or more settings with the network computing device 340 of Figure 5. In one embodiment, the mechatronic device 202 receives configuration or calibration information related to a user of the particular mechatronic device 202. In another embodiment, the mechatronic device 202 sends configuration or calibration data to the network computing device 340. In one embodiment, the synchronized configuration and calibration data includes any of the data, discussed above, that is sent over the BDB 120. In addition, the synchronized data may include any other configuration or calibration data used by the mechatronic device 120.

In one embodiment, synchronizing the data includes determining the differences between data on the mechatronic device 202 and data associated with the particular mechatronic device 202 on the network computing device 340, and sending that data from one device to the other. In one embodiment, the network computing device 340 stores the data associated with the mechatronic device 202 in a database in association with data identifying the particular mechatronic device, e.g., a serial number. In one embodiment, when the particular mechatronic device 202 is synchronized again, the network computing device 340 determines the differences in the data based on the data in the database. In one embodiment, after determining which control data is different, the mechatronic device 202 sends control data to the network computing device 340 that overwrites control data associated with the mechatronic device 202. In another embodiment, the network computing device 340 sends control data to the mechatronic device 202 that overwrites such data on the mechatronic device. In one embodiment, some data is sent both ways for overwriting. Whether the control data is sent to or from the mechatronic device 202 may be based on one or more methods. For example, in one embodiment, time stamps are associated with the data so that the newest data associated with a particular item of control data is saved on both the mechatronic device 202 and the network computing device 340. In other embodiments, predetermined rules regarding particular items of control data determine how the data is synchronized. In one embodiment, a selection by the user of the device, or a selection by a prosthetist determines in which data particular items of control data are synchronized. In one embodiment, a new mechatronic device 202 receives initial control data from a database associated with the network computing device 340 that stores initial data or overwrites any existing data on the mechatronic device 202.

In one embodiment, the network computing device 340 acts as a conduit to send and receive the configuration or calibration data to another network computing device 341 that stores the data. In one embodiment, the network computing device 340 is a PDA or mobile telephone that communicates with the mechatronic device 202 via a short range network and relays that data to the network computing device 341. In one such embodiment, the network computing device 341 includes a server computer. Thus, the mechatronic device 202 may synchronize configuration and calibration data with one or both of the network computing devices 340 and 341.

Next at a block 630, the mechatronic device 202 stores any received data. Also, or alternatively, the network computing devices 340 and 341 store any received data. In one embodiment, one or more of the devices 202, 340, or 341 also store data related to the synchronization, e.g., a timestamp or data identifying the devices or data involved in the synchronization. In one embodiment, the network computing device 340 or 341 stores the data in a database in association with the mechatronic device. Returning to Figure 6, the method 600 proceeds to an end state.

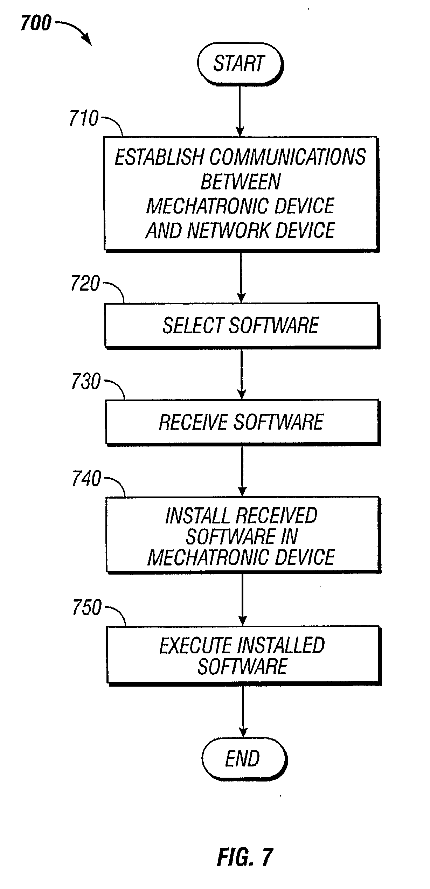

Figure 7 is a flowchart illustrating one embodiment of a method 700 of installing, replacing, augmenting, or deinstalling software on the mechatronic device. The method 700 begins at a block 710 in which the mechatronic device 202 establishes communication with a source device containing software configured to execute on the mechatronic device 202. In one embodiment, the source device includes the network computing device 340. In such an embodiment, the mechatronic device 202 establishes communications with the network computing device 340 via the network 350. In another embodiment, the source device also includes the network computing device 341. In such an embodiment, the mechatronic device establishes communications with the network computing device 341 through the networks 350 and 351 via the network computing device 340. In one embodiment, the source device includes another mechatronic device. In another embodiment, the source device includes a storage card in communication with the storage card interface 366. The software could be low level firmware and/or high level software, for example.

Moving to a block 720, the mechatronic device 202 or the user of the device 202 selects software to be installed thereon. In one embodiment, the user selects from a list of software adapted to various activities, e.g., hiking, biking, or jogging. In one embodiment, the list is displayed on a user interface associated with the network computing device 340. In one embodiment, the user interface includes a web browser. In one such embodiment, the user interface receives the list from the network computing device 341.

Proceeding to a block 730, the mechatronic device 202 receives the software from the source device. In one embodiment, receiving the software includes transferring then software over the network 350. In another embodiment, receiving the software includes having a storage card installed in the storage card interface 366.

Next at a block 740, the mechatronic device 202 installs the software for execution. Installing the software may include saving the software to a portion of the memory 362, updating pointers or jump tables in the memory 362 to replace or augment previously installed software, or storing a record of the software installation. In one embodiment, the record includes sufficient data to remove the newly installed software. In one embodiment, the mechatronic device 202 saves the received software to its memory 362. In another embodiment, the mechatronic device 202 executes the new software directly from a storage card.

Moving to a block 750, the mechatronic device executes the new software. The new software may replace all or a portion of one or more of the state machine module 210, the hardware abstraction module 212, the dynamic learning module 214, a configuration module 216, the BDB module 218, or any other suitable software module of the mechatronic device 202. The new software may include software updates to fix bugs, improve performance, or provide additional features. In one embodiment, the new software may include instructions for controlling the mechatronic device 202 to perform one or more specific activities such as hiking, biking, swimming, jogging, throwing, jumping, or for movement over a particular type of terrain.

It is to be appreciated that depending on the embodiment, certain acts or events of a method described herein can be performed in a different sequence, may be added, merged, or left out all together (e.g., not all described acts or events are necessary for the practice of the method). Moreover, in certain embodiments, acts or events may be performed concurrently, e.g., through multi-threaded processing, interrupt processing, or multiple processors, rather than sequentially.

While the above detailed description has shown, described, and pointed out novel features of the invention as applied to various embodiments, it will be understood that various omissions, substitutions, and changes in the form and details of the device or process illustrated may be made by those skilled in the art without departing from the spirit of the invention. As will be recognized, the present invention may be embodied within a form that does not provide all of the features and benefits set forth herein, as some features may be used or practiced separately from others.
1. A system for controlling motion of a device associated with a limb, the system comprising:
   a plurality of mechatronic devices, wherein each of the plurality of mechatronic devices is configured to communicate data with at least one other of the plurality of mechatronic devices, and wherein at least one of the mechatronic device controls an actuator.
2. The system of clause 1, wherein at least one of the plurality of mechatronic devices is configured to generate a control state for at least one other of the plurality of mechatronic devices based on the communicated data.
3. The system of clause 1, wherein the communicated data is used to synchronize the mechatronic devices.
4. The system of clause 1, wherein each of the plurality of mechatronic devices comprises an artificial joint.
5. The system of clause 1, wherein at least one of the plurality of mechatronic devices comprises a prosthetic knee and at least one of the plurality of mechatronic devices comprises a prosthetic ankle.
6. The system of clause 1, wherein the device associated with a limb is an artificial leg.
7. A mechatronic device for controlling motion of a human limb in cooperation with at least one other mechatronic device, the device comprising:
   a communication interface configured to communicate data with the at least one other mechatronic device;
   a sensor configured to obtain a value indicative of at least one motion parameter of the limb;
   an actuator configured to affect the at least one motion parameter of the limb; and
   a processor configured to control the actuator based on the received communicated data and the at least one motion parameter.
8. The mechatronic device of clause 7, wherein the communicated data comprises the parameter value obtained by the sensor.
9. The mechatronic device of clause 7, wherein the communicated data comprises state machine data received from the other mechatronic device.
10. The mechatronic device of clause 7, wherein the communicated data comprises configuration data received from the other mechatronic device.
11. A mechatronic device for controlling motion of a device associated with a limb in cooperation with at least one other mechatronic device, the device comprising:
   a communication interface configured to communicate data with the at least one other mechatronic device;
   a processor configured to generate a control state of the at least one other mechatronic device and to communicate data associated with the control state through the communication interface; and
   an actuator controlled by the processor so as to effectuate movement of the human limb.
12. The mechatronic device of clause 11, wherein the communicated data comprises data obtained by at least one sensor of the other mechatronic device.
13. The mechatronic device of clause 11, wherein the communicated data comprises configuration data received from the other mechatronic device.
14. The mechatronic device of clause 11, wherein the processor is further configured to determine at least one actuator control command based on the control state, and wherein the communicated data comprises the at least one actuator control command.
15. The mechatronic device of clause 11, wherein the communicated data comprises software that when executed by the processor affects the selection of the control state.
16. A method of synchronizing a first mechatronic device with a second mechatronic device associated with a device associated with a limb, the method comprising:
   communicating data between the second mechatronic device and the first mechatronic device;
   generating a control state on the first mechatronic device for the second mechatronic device in response to the communicated data;
   communicating the control state to the second mechatronic device; and
   controlling an actuator on the second mechatronic device based at least in part on the control state.
17. The method of clause 16, further comprising generating a command to control an actuator of the second mechatronic device in response the control state.
18. The method of clause 16, further comprising generating a command to control an actuator of the first mechatronic device in response to the communicated data.
19. The method of clause 16, wherein the communicated data includes sensor data received from the second mechatronic device.
20. The method of clause 16, wherein the communicated data includes at least a portion of the information indicative of the control state.
21. The method of clause 16, wherein the communicated data includes computer software and wherein the control state is generated on the first mechatronic device by executing the computer software.
22. A system for controlling motion of a device associated with a limb, the system comprising:
   a mechatronic device;
   a sensor associated with a human limb which provides motion parameter data to the mechatronic device, wherein the mechatronic device uses the motion parameter data for synchronization.
23. The system of clause 22, wherein the sensor receives signals from the human nervous system.
24. The system of clause 22, wherein the sensor receives signals from sensors associated with a sound limb.
25. The system of clause 22, wherein the motion parameter data is used for synchronization with another mechatronic device.
26. The system of clause 25, wherein the other mechatronic device provides motion parameter data to the mechatronic device.
27. A mechatronic system attached to a human body, the system comprising:
   a sensor configured to provide data indicative of movement of the human body;
   an actuator configured to control movement of at least a portion of the human body;
   a processor configured to execute instructions configured to control the actuator based on the sensor data; and
   a communication interface configured to communicate data with a data source
   wherein the processor is configured to receive at least a portion of the instructions from the data source.
28. The system of clause 27, wherein the received instructions, when executed, adapt the mechatronic system for a particular activity.
29. The system of clause 28, wherein the particular activity comprises at least one of hiking, jogging, and running.
30. The system of clause 27, wherein the data source comprises a computing device.
31. The system of clause27, wherein the data source comprises a storage card.
32. The system of clause 27, wherein the actuator is configured to control movement of a knee of the body.
33. The system of clause 27, wherein the instructions comprises a first state machine.
34. The system of clause 33, wherein the received instructions comprise a second state machine that replaces at least a portion of the first state machine.
35. The system of clause 27, wherein the communications interface is configured to communicate data using an Internet Protocol.
36. The system of clause 27, wherein the communications interface is configured to communicate data over a wireless communications link.
37. The system of clause 27, wherein the source device comprises first and second computing devices and wherein the communications interface is configured to communicate over a first network to the first computing device and wherein the first computing device is configured to communicate with the second computing device over a second network.
38. The system of clause 37, wherein the processor is configured to receive the instructions from the second communications device.
39. A method of synchronizing a computing device with a mechatronic system associated with a human limb, the method comprising:
   communicating data between the mechatronic system and the computing device;
   storing the data on the computing device;
   generating a control state on the mechatronic system in response to the data; and
   controlling an actuator on the second mechatronic system based at least in part on the control state.
40. The method of clause 39, further comprising:
   receiving sensor data on the mechatronic system; and
   generating the data to be communicated on the mechatronic system in response to the sensor data.
41. The method of clause 40, wherein communicating data between the mechatronic system and the computing device comprises:
   sending data from the mechatronic system to the computing device; and
   sending data from the computing device to the mechatronic system.
42. The method of clause 39, further comprising:
   retrieving data from a database based on information associated with the mechatronic system;
   communicated the retrieved data from the computing device to the mechatronic system; and
   storing the retrieved data on the mechatronic system.
43. The method of clause 39, wherein storing the retrieved data on the computing device comprises:
   associating the data with data indicative of the mechatronic system; and
   storing the data and the data indicative of the mechatronic system in a database.
44. A mechatronic system attached to a human body, the system comprising:
   means for providing data indicative of movement of the human body;
   means for controlling movement of at least a portion of the human body;
   means for storing instructions configured to control the actuator based on the sensor data;
   means for executing said stored instructions and controlling the actuator based on the sensor data; and
   means for communicating data with a data source and receiving at least a portion of the instructions from the data source into said means for storing.
45. The system of clause 44, wherein the received instructions, when executed, adapt the mechatronic system for a particular activity.
46. The system of clause 46, wherein the particular activity comprises at least one of hiking, jogging, and running.
47. The system of clause 44, wherein means for controlling comprising means for controlling movement of a knee of the body.

## Claims

1. A mechatronic system attachable to a human body, the system comprising:
a sensor configured to provide data indicative of movement of the human body;
an actuator configured to affect at least one motion parameter of a mechatronic device attachable to the human body;
a processor configured to execute instructions configured to control the actuator based on the sensor data; and
a communication interface configured to communicate data with a data source;
wherein the processor is configured to receive at least a portion of the instructions from the data source.

2. The mechatronic system of Claim 1 wherein the mechatronic device comprises a prosthetic joint.

3. The mechatronic system of Claim 2, wherein the sensor is provided on the prosthetic joint.

4. The mechatronic system of any one of Claims 1 to 3, wherein the data source is external to the mechatronic device.

5. The mechatronic system of any one of Claims 1 to 4, further comprising a memory configured to store instructions for controlling the actuator based on sensor data.

6. The system of any one of Claims 1 to 5, wherein the received instructions, when executed, adapt the mechatronic system for a particular activity, the activity comprising at least one of hiking, jogging, and running.

7. The system of any one of Claims 1 to 6, wherein the data source comprises a computing device or a storage card.

8. The system of Claim 5, wherein the received instructions comprise a second state machine that replaces at least a portion of a first state machine stored in the memory.

9. The system of any one of Claims 1 to 8, wherein the communication interface is configured to communicate data using an Internet Protocol or a wireless communications link.

10. The system of any one of Claims 1 to 9, wherein the data source comprises first and second computing devices and wherein the communication interface is configured to communicate over a first network to the first computing device and wherein the first computing device is configured to communicate with the second computing device over a second network.

11. A method of synchronizing a computing device with a mechatronic system associated with a human limb, the method comprising:
communicating data between the mechatronic system and the computing device;
storing the data on the computing device;
generating a control state on the mechatronic system in response to the data; and
controlling an actuator on the mechatronic system based at least in part on the control state.

12. The method of Claim 11, further comprising:
determining differences between data on the mechatronic system and data associated with the mechatronic system on the computing device;
updating data on one or both of the mechatronic system and computing device based on said differences;
storing the updated data on one or both of the mechatronic system and computing device.

13. The method of Claim 11 or 12, further comprising:
receiving sensor data on the mechatronic system; and
generating the data to be communicated to the computing device on the mechatronic system in response to the sensor data.

14. The method of any one of Claims 11 to 13, wherein communicating data between the mechatronic system and the computing device comprises:
sending data from the mechatronic system to the computing device; and
sending data from the computing device to the mechatronic system, and / or wherein storing the data on the computing device comprises:
associating the data with data indicative of the mechatronic system; and
storing the data and the data indicative of the mechatronic system in a database.

15. The method of any one of Claims 11 to 14, further comprising:
retrieving data from a database on the computing device based on information associated with the mechatronic system;
communicating the retrieved data from the computing device to the mechatronic system; and
storing the retrieved data on the mechatronic system.
